# EUROPEAN PATENT SPECIFICATION

(11) **EP 3 031 070 B1**
(45) Date of publication and mention of the grant of the patent: **30.12.2020**
(21) Application number: 14835047.3
(22) Date of filing: 07.08.2014
(51) Int. Cl.: H01J 49/00

(54) **SYSTEMS AND METHODS FOR RECORDING AVERAGE ION RESPONSE**
SYSTEME UND VERFAHREN ZUR AUFZEICHNUNG EINER DURCHSCHNITTLICHEN IONENREAKTION
SYSTÈMES ET PROCÉDÉS D'ENREGISTREMENT DE RÉPONSE MOYENNE D'ION

(30) Priority: 09.08.2013 US 201361863940 P
(43) Date of publication of application: 15.06.2016
(73) Proprietor: DH Technologies Development PTE. Ltd., Singapore 739256 (SG)
(72) Inventor: BLOOMFIELD, Nic, G., Newmarket, Ontario L3K 2K4 (CA); LOBODA, Alexandre, V., Thornhill, ON L4J 5G7 (CA); CHERNUSHEVICH, Igor, Toronto, ON M2R 3K7 (CA)
(74) Representative: J A Kemp LLP
(86) International application number: PCT/IB2014/001477
(87) International publication number: WO 2015/019163

(56) References cited:
- WO-A2-2006/090138
- US-A- 6 094 627
- US-A1- 2005 161 595
- US-A1- 2011 049 355
- US-A1- 2011 049 355
- US-A1- 2011 186 727
- US-A1- 2011 186 727
- US-A1- 2012 228 488

## Description

Often when mass spectra are recorded, the information contained in the spectra is insufficient to identify the ion species. One example of a case of insufficient information is the lack of knowledge of how many charges an ion is carrying. Without the knowledge of the charge, correct mass assignment can be difficult. Another problem can arise when someone is trying to decide if a peak in a spectrum belongs to one class of compounds (for instance, lipids) or another class of compounds (for instance, peptides). There is almost always a benefit to collecting complementary information about the sample under investigation, especially if the cost of collecting this information is not that high.

The document WO 2006/090138 A2 discloses a method of correcting for deadtime effects based on total ion counts and Poisson statistics.

The invention is defined in claims. A system is disclosed for calculating and storing an average amplitude response for each peak of a mass spectrum during data acquisition. A mass analyzer that includes an analog-to-digital converter (ADC) detector subsystem analyzes a beam of ions produced by an ion source that ionizes sample molecules. A processor instructs the mass analyzer to analyze N extractions of the ion beam, producing N sub-spectra. For each sub-spectrum of the N sub-spectra, the processor counts a nonzero amplitude from the ADC detector subsystem as one ion. As a result, a count of one for each ion of each sub-spectrum is produced. The processor sums the ADC amplitudes and counts of the N sub-spectra.

A spectrum is produced that includes a summed ADC amplitude and a total count for each ion of the spectrum. For each ion of the spectrum, the processor calculates an estimated ion count from a Poisson distribution of the total count of each ion for the N sub-spectra. For each ion of the spectrum, the processor calculates and stores an average amplitude response by dividing the summed amplitude by the estimated ion count. Finally, the processor instructs the mass analyzer to perform another series of N extractions of the sample that produces another N sub-spectra, and the entire process is started again.

A method is disclosed for calculating and storing an average amplitude response for each peak of a mass spectrum during data acquisition. A mass analyzer that includes an ADC detector subsystem is instructed to analyze N extractions of an ion beam using a processor. N sub-spectra are produced. For each sub-spectrum of the N sub-spectra, a nonzero amplitude from the ADC detector subsystem is counted as one ion using the processor. A count of one is produced for each ion of each sub-spectrum. The ADC amplitudes and counts of the N sub-spectra are summed using the processor.

A spectrum is produced that includes a summed ADC amplitude and a total count for each ion of the spectrum. For each ion of the spectrum, an estimated ion count is calculated from a Poisson distribution of the total count of each ion for the N sub-spectra using the processor. For each ion of the spectrum, an average amplitude response is calculated by dividing the summed amplitude by the estimated ion count and stored using the processor. Finally, the mass analyzer is instructed again to perform another series of N extractions of a sample using a processor.

A computer program product is disclosed that includes a non-transitory and tangible computer-readable storage medium whose contents include a program with instructions being executed on a processor so as to perform a method for calculating and storing an average amplitude response for each peak of a mass spectrum during data acquisition. In various embodiments, the method includes providing a system, wherein the system comprises one or more distinct software modules, and wherein the distinct software modules comprise a control module and an analysis module.

The control module instructs a mass analyzer that includes an ADC detector subsystem to analyze N extractions of an ion beam. N sub-spectra are produced. For each sub-spectrum of the N sub-spectra, the analysis module counts a nonzero amplitude from the ADC detector subsystem as one ion. A count of one for each ion of each sub-spectrum is produced. The analysis module sums the ADC amplitudes and counts of the N sub-spectra.

A spectrum is produced that includes a summed ADC amplitude and a total count for each ion of the spectrum. For each ion of the spectrum, the analysis module calculates an estimated ion count from a Poisson distribution of the total count of each ion for the N sub-spectra. For each ion of the spectrum, the analysis module calculates and stores an average amplitude response by dividing the summed amplitude by the estimated ion count using the analysis module. Finally, the control module instructs the mass analyzer to perform another series of N extractions of the sample that producing another N sub-spectra, and the entire process is started again.

These and other features of the applicant's teachings are set forth herein.

The skilled artisan will understand that the drawings, described below, are for illustration purposes only. The drawings are not intended to limit the scope of the present teachings in any way.
Figure 1 is a block diagram that illustrates a computer system, in accordance with various embodiments.
Figure 2 is an exemplary diagram of a time-of-flight (TOF) mass spectrometry system showing ions entering a TOF tube, in accordance with various embodiments.
Figure 3 is a plot of sub-spectra received by the processor of Figure 2 for a series of N extractions, in accordance with various embodiments.
Figure 4 is a plot of the analog-to-digital converter (ADC) spectrum produced by the processor of Figure 2 from summing the N sub-spectra of Figure 3, in accordance with various embodiments.
Figure 5 is an exemplary flowchart showing a method for calculating and storing an average amplitude response for each peak of a mass spectrum during data acquisition, in accordance with various embodiments.
Figure 6 is a schematic diagram of a system that includes one or more distinct software modules that performs a method for calculating and storing an average amplitude response for each peak of a mass spectrum during data acquisition, in accordance with various embodiments.

Before one or more embodiments of the present teachings are described in detail, one skilled in the art will appreciate that the present teachings are not limited in their application to the details of construction, the arrangements of components, and the arrangement of steps set forth in the following detailed description or illustrated in the drawings. Also, it is to be understood that the phraseology and terminology used herein is for the purpose of description and should not be regarded as limiting.

### COMPUTER-IMPLEMENTED SYSTEM

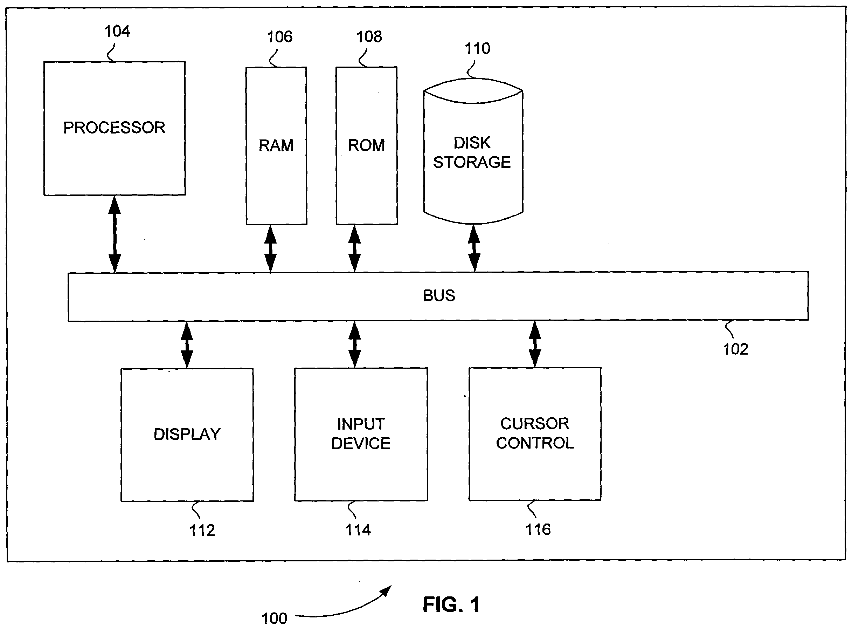

Figure 1 is a block diagram that illustrates a computer system 100, in accordance with various embodiments. Computer system 100 includes a bus 102 or other communication mechanism for communicating information, and a processor 104 coupled with bus 102 for processing information. Computer system 100 also includes a memory 106, which can be a random access memory (RAM) or other dynamic storage device, coupled to bus 102 for storing instructions to be executed by processor 104. Memory 106 also may be used for storing temporary variables or other intermediate information during execution of instructions to be executed by processor 104. Computer system 100 further includes a read only memory (ROM) 108 or other static storage device coupled to bus 102 for storing static information and instructions for processor 104. A storage device 110, such as a magnetic disk or optical disk, is provided and coupled to bus 102 for storing information and instructions.

Computer system 100 may be coupled via bus 102 to a display 112, such as a cathode ray tube (CRT) or liquid crystal display (LCD), for displaying information to a computer user. An input device 114, including alphanumeric and other keys, is coupled to bus 102 for communicating information and command selections to processor 104. Another type of user input device is cursor control 116, such as a mouse, a trackball or cursor direction keys for communicating direction information and command selections to processor 104 and for controlling cursor movement on display 112. This input device typically has two degrees of freedom in two axes, a first axis (*i.e.,* x) and a second axis (*i.e.,* y), that allows the device to specify positions in a plane.

A computer system 100 can perform the present teachings. Consistent with certain implementations of the present teachings, results are provided by computer system 100 in response to processor 104 executing one or more sequences of one or more instructions contained in memory 106. Such instructions may be read into memory 106 from another computer-readable medium, such as storage device 110. Execution of the sequences of instructions contained in memory 106 causes processor 104 to perform the process described herein. Alternatively hard-wired circuitry may be used in place of or in combination with software instructions to implement the present teachings. Thus implementations of the present teachings are not limited to any specific combination of hardware circuitry and software.

The term "computer-readable medium" as used herein refers to any media that participates in providing instructions to processor 104 for execution. Such a medium may take many forms, including but not limited to, non-volatile media, volatile media, and transmission media. Non-volatile media includes, for example, optical or magnetic disks, such as storage device 110. Volatile media includes dynamic memory, such as memory 106. Transmission media includes coaxial cables, copper wire, and fiber optics, including the wires that comprise bus 102.

Common forms of computer-readable media include, for example, a floppy disk, a flexible disk, hard disk, magnetic tape, or any other magnetic medium, a CD-ROM, digital video disc (DVD), a Blu-ray Disc, any other optical medium, a thumb drive, a memory card, a RAM, PROM, and EPROM, a FLASH-EPROM, any other memory chip or cartridge, or any other tangible medium from which a computer can read.

Various forms of computer readable media may be involved in carrying one or more sequences of one or more instructions to processor 104 for execution. For example, the instructions may initially be carried on the magnetic disk of a remote computer. The remote computer can load the instructions into its dynamic memory and send the instructions over a telephone line using a modem. A modem local to computer system 100 can receive the data on the telephone line and use an infra-red transmitter to convert the data to an infra-red signal. An infra-red detector coupled to bus 102 can receive the data carried in the infra-red signal and place the data on bus 102. Bus 102 carries the data to memory 106, from which processor 104 retrieves and executes the instructions. The instructions received by memory 106 may optionally be stored on storage device 110 either before or after execution by processor 104.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

The following descriptions of various implementations of the present teachings have been presented for purposes of illustration and description. It is not exhaustive and does not limit the present teachings to the precise form disclosed. Modifications and variations are possible in light of the above teachings or may be acquired from practicing of the present teachings. Additionally, the described implementation includes software but the present teachings may be implemented as a combination of hardware and software or in hardware alone. The present teachings may be implemented with both object-oriented and non-object-oriented programming systems.

### SYSTEMS AND METHODS FOR RECORDING AVERAGE ION RESPONSE

As described above, often when mass spectra are recorded, the information contained in the spectra is insufficient to identify the ion species. There is almost always a benefit to collecting complementary information about the sample under investigation, especially if the cost of collecting this information is not that high.

In various embodiments, an average ion response is measured for each ion peak in a recorded spectrum in order to provide complementary information about the sample under investigation. This complementary information about the ion response is used as a differentiating mechanism. In the case of a quadrupole mass spectrometer or an ion trap mass spectrometer, ions are commonly detected as a stream of individual ions. In one embodiment, the amplitude of each ion response pulse can be recorded with a high speed digitizer. Average response can be calculated based on the measurements from individual ion pulses. In an alternative embodiment, the average amplitude from many ion pulses can be recorded by integrating a total charge generated by the detector and dividing it by the number of ion pulses recorded. In both embodiments, for each data point on the mass-to-charge (m/z) scale, an average pulse amplitude can be stored together with a mass, intensity pair.

In the case of a time-of-flight (TOF) mass analyzer/detector, recording an average ion response can be more complicated. At lower ion currents individual ion events can be counted. For each ion event, an intensity of the pulse can be measured and recorded. Therefore, an average ion response can be measured for each data point on the m/z time scale. When the ion flux increases, multiple ions can be arriving at the same time or nearly at the same time (within a response time of the detector). Still, the data acquisition system can record those events and derive an estimate of the average pulse response for each m/z data point.

In liquid chromatography couple mass spectrometry (LCMS) applications, this information can be further enhanced by following the liquid chromatography (LC) profile for individual peaks (or data points) in mass spectrum. In time-of-flight mass spectrometry (TOF-MS), the LC profile of the average response for a given ion can provide an estimate of how many ions are arriving simultaneously during the LC peak of the given ion. If detector saturation is skewing the measurement of the ion response then proper correction algorithms can be applied to restore the ion intensity recording by correcting it to the non-saturated conditions. An average ion response at a given point on m/z scale can be used to recalculate the average ion response for ions of a given peak in the mass spectrum. Thus, after running a peak finding routine, the user is presented with a mass, intensity, and average response triplet. Peak finding and noise-filtering can also be enabled based on the requirement of the average response to fit into a certain window.

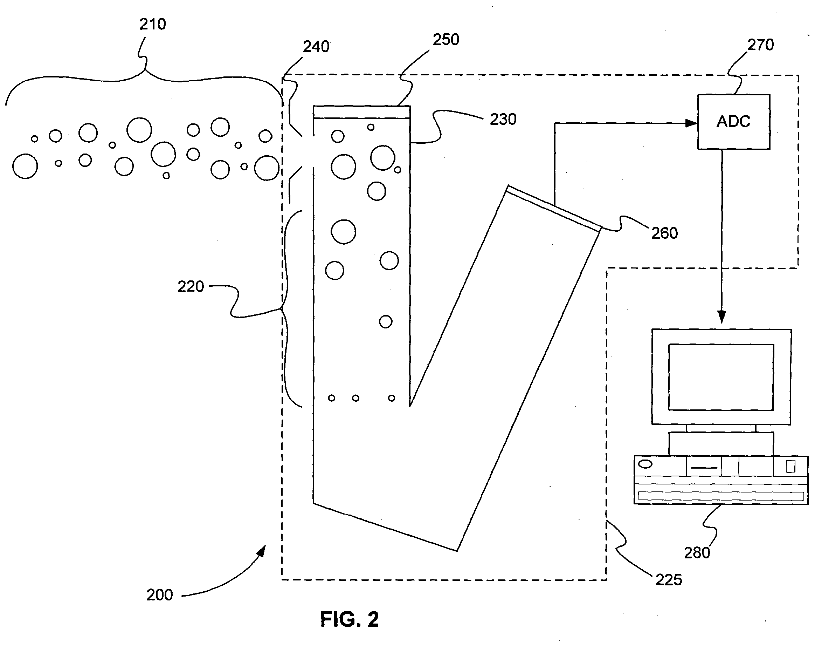

Figure 2 is an exemplary diagram of a time-of-flight (TOF) mass spectrometry system 200 showing ions 210 entering TOF tube 230, in accordance with various embodiments. TOF mass spectrometry system 200 includes TOF mass analyzer 225 and processor 280. TOF mass analyzer 225 includes TOF tube 230, skimmer 240, extraction device 250, ion detector 260, and ADC detector subsystem 270. Skimmer 240 controls the number of ions entering TOF tube 230. Ions 210 are moving from an ion source (not shown) to TOF tube 230. The number of ions entering TOF tube 230 can be controlled by pulsing skimmer 240, for example.

Extraction device 250 imparts a constant energy to the ions that have entered TOF tube 230 through skimmer 240. Extraction device 250 imparts this constant energy by applying a fixed voltage at a fixed frequency, producing a series of extraction pulses, for example. Because each ion receives the same energy from extraction device 250, the velocity of each ion depends on its mass. According to the equation for kinetic energy, velocity is proportional to the inverse square root of the mass. As a result, lighter ions fly through TOF tube 230 much faster than heavier ions. Ions 220 are imparted with a constant energy in a single extraction, but fly through TOF tube 230 at different velocities.

Time is needed between extraction pulses to separate the ions in TOF tube 230 and detect them at ion detector 260. Enough time is allowed between extraction pulses so that the heaviest ion can be detected.

Ion detector 260 generates an electrical detection pulse for every ion that strikes it during an extraction. These detection pulses are passed to ADC detector subsystem 270, which records the amplitudes of the detected pulses digitally. In a TDC detector subsystem, for example, ADC detector subsystem 270 is replaced by a constant fraction discriminator (CFD) coupled to a TDC. The CFD removes noise by only transmitting pulses that exceed a threshold value, and the TDC records the time values at which the electrical detection pulses occur.

Processor 280 receives the pulses recorded by ADC detector subsystem 270 during each extraction. Because each extraction may contain only a few ions from a compound of interest, the responses for each extraction can be thought of as a sub-spectrum. In order to produce more useful results, processor 280 can sum the sub-spectra of time values from a number of extractions to produce a full spectrum.

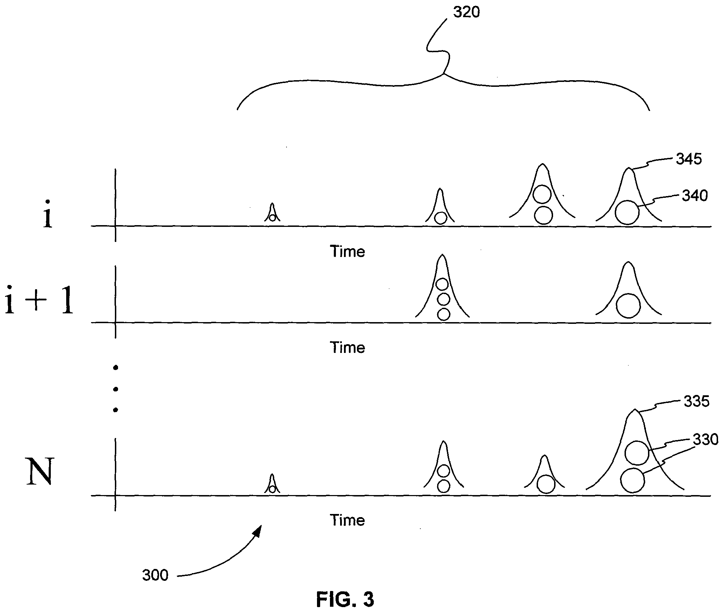

Figure 3 is a plot of sub-spectra 300 received by processor 280 of Figure 2 for a series of N extractions, in accordance with various embodiments. Sub-spectra for extractions i through N include time values for each ion detected. The horizontal position of each ion in each sub-spectrum represents the time it takes that ion to be detected relative to the extraction pulse. Ions 320 of extraction i in Figure 3 correspond to ions 220 in Figure 2, for example.

As shown in sub-spectra 300 of Figure 3, an ADC produces an amplitude response that is dependent on the number of ions hitting the detector at substantially the same time. For example, the two ions 330 in extraction N produce amplitude response 335 that is larger than amplitude response 345, which is produced by a single ion 340 in extraction i. In other words, the response that an ADC produces is proportional to the number of ions hitting the detector at substantially the same time.

A TDC, on the other hand, does not record a signal that is proportional to the number of ions hitting the detector at substantially the same time. Instead, a TDC records only if at least one ion of a particular mass impacted the detector.

TDC information, however, can be determined from ADC information. For example, in sub-spectra 300 of Figure 3, a processor, such as processor 280 of Figure 2 can count the impact of the two ions 330 as a single ion hit for extraction N. In other words, for every extraction, in addition to the ADC response, a single hit is recorded for any amplitude response for a given mass. This produces a TDC equivalent response. A ratio of the ADC response to the number of ions is then determined from both the ADC response and the equivalent TDC response.

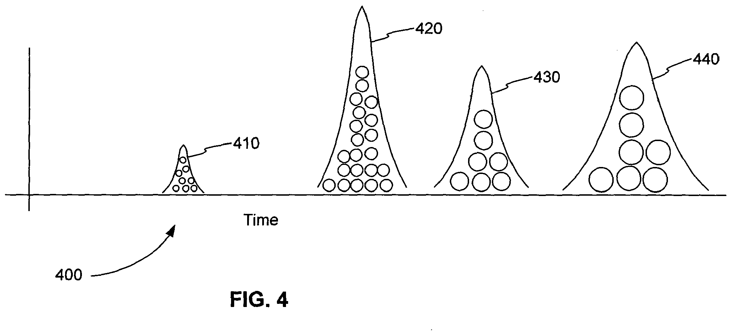

Figure 4 is a plot of the ADC spectrum 400 produced by processor 280 of Figure 2 from summing the N sub-spectra of Figure 3, in accordance with various embodiments. As described above, in various embodiments an average ion amplitude response is recorded for each ion peak in a spectrum. Spectrum 400 includes four different ion peaks 410, 420, 430, and 440. Ion peak 440, for example, represents the summation of the amplitudes recorded for a specific ion hitting the detector over N extractions. In order to determine the average amplitude response for this specific ion, the amplitude of ion peak 440 needs to be divided by the number of those specific ions that hit the detector.

Determining the number of those specific ions that hit the detector is complicated by the possibility of more than one ion hitting the detector at any one time. For example, as shown in Figure 3, one of the amplitudes that makes up the amplitude of ion peak 440 is amplitude 335. Amplitude 335 is the result of two ions 330 hitting the detector at the same time in extraction N.

In various embodiments, the number or count of specific ions that produced each peak in a spectrum is calculated from a Poisson distribution of the equivalent TDC ion count K for N extractions. As long as there are a number of extractions above a certain threshold level where there are no ions hitting the detector, a Poisson distribution can be used to estimate the ion count for a peak. For example, as long as 10% of the extractions do not measure an amplitude for a specific ion, a Poisson distribution can be used to calculate the ion count for that specific ion.

As a result, the average amplitude response for the ion represented by ion peak 440 is found by dividing the amplitude of ion peak 440 by the ion count calculated from a Poisson distribution of the equivalent TDC ion count K recorded over N extractions. This average amplitude response or ratio of amplitude with respect to ion count varies for compounds with different charges and for compounds of different classes. As a result, the average amplitude response can be calculated and stored for every peak and used to differentiate peaks in addition to mass and intensity.

Although the examples described above and in Figures 2-4 are directed to a TOF mass analyzer, the methods described herein also apply to other types of mass analyzers including, but not limited to, quadrupoles and ion traps. Essentially, any mass analyzer that produces a stream of analog pulses, which can be counted and have an amplitude dependent on intensity, can be used to record an average amplitude response.

In various embodiments, a mass analyzer calculates and stores the average amplitude response for each ion peak in a spectrum in addition to the mass and the intensity. These three values are calculated and stored in real-time during acquisition. The stored average amplitude responses of the peaks of a spectrum can then be used in post-acquisition analyses, for example.

### System for Calculating and Storing an Average Amplitude Response

Returning to Figure 2, system 200 is an exemplary mass spectrometry system for calculating and storing an average amplitude response for each peak of a mass spectrum during data acquisition. As described above, system 200 includes mass analyzer 225 and processor 280. Mass analyzer 225 is shown in Figure 2 as a time-of-flight mass analyzer. Mass analyzer 225, however, can be any mass analyzer that produces a stream of analog pulses, which can be counted and have an amplitude dependent on intensity. Mass analyzer 225 can also be a quadrupole or an ion trap, for example.

Mass analyzer 225 can be coupled to one or more mass spectrometry components (not shown) in system 200. One or more mass spectrometry components can include, but are not limited to, quadrupoles, for example. Mass analyzer 225 can also be coupled to one or more additional mass analyzers.

Mass spectrometry system 200 can also include one or more separation devices (not shown). The separation device can perform a separation technique that includes, but is not limited to, liquid chromatography, gas chromatography, capillary electrophoresis, or ion mobility. Mass analyzer 225 can include separating mass spectrometry stages or steps in space or time, respectively.

Processor 280 can be, but is not limited to, a computer, microprocessor, or any device capable of sending and receiving control signals and data to and from mass analyzer 225 and processing data. Processor 280 is, for example, a computer system such as the computer system shown in Figure 1. Processor 280 is in communication with mass analyzer 225.

Mass analyzer 225 includes ADC detector subsystem 270. Mass analyzer 225 analyzes a beam of ions 210, for example. The beam of ions is produced by an ion source (not shown) that ionizes sample molecules, for example.

Processor 280 instructs mass analyzer 225 to analzye N extractions of the ion beam, producing N sub-spectra. For each sub-spectrum of the N sub-spectra, processor 280 counts a nonzero amplitude from the ADC detector subsystem as one ion. As a result, a count of one for each ion of each sub-spectrum is produced. Processor 280 sums the amplitudes and counts of the N sub-spectra. A spectrum is produced that includes a summed ADC amplitude and a total count for each ion of the spectrum. The total count is, for example, a TDC equivalent count. For each ion of the spectrum, processor 280 calculates an estimated ion count from a Poisson distribution of the total count of each ion for the N sub-spectra. For each ion of the spectrum, processor 280 calculates and stores an average amplitude response by dividing the summed amplitude by the estimated ion count. Finally, processor 280 instructs mass analyzer 225 to perform another series of N extractions of the sample that produces another N sub-spectra, and the entire process is started again.

In various embodiments, mass analyzer 225 further includes a TDC detector subsystem. As a result, for each sub-spectrum of the N sub-spectra, processor 280 counts a nonzero amplitude from the ADC detector subsystem as one ion by reading the TDC detector subsystem.

In various embodiments, processor 280 calculates an estimated ion count from a Poisson distribution of the total count for the N sub-spectra, if N exceeds the total count by a threshold level. If N does not exceed the total count by the threshold level, the Poisson distribution results are unreliable. In other words, the threshold level is used to insure that the Poisson distribution provides reliable results. In order for Poisson statistics to provide a reliable total ion count, the Poisson distribution needs to have a minimum number of extractions that do not include the ion of interest. This minimum number of extractions that do not include the ion of interest is the threshold level, for example.

In various embodiments, for each ion of the spectrum, an average amplitude response of an ion of the spectrum is used to distinguish the ion from another ion with same mass but a different charge. In various alternative embodiments, an average amplitude response of an ion of the spectrum is used to distinguish the ion from another ion with same mass but from a different class of compounds.

### Method for Calculating and Storing an Average Amplitude Response

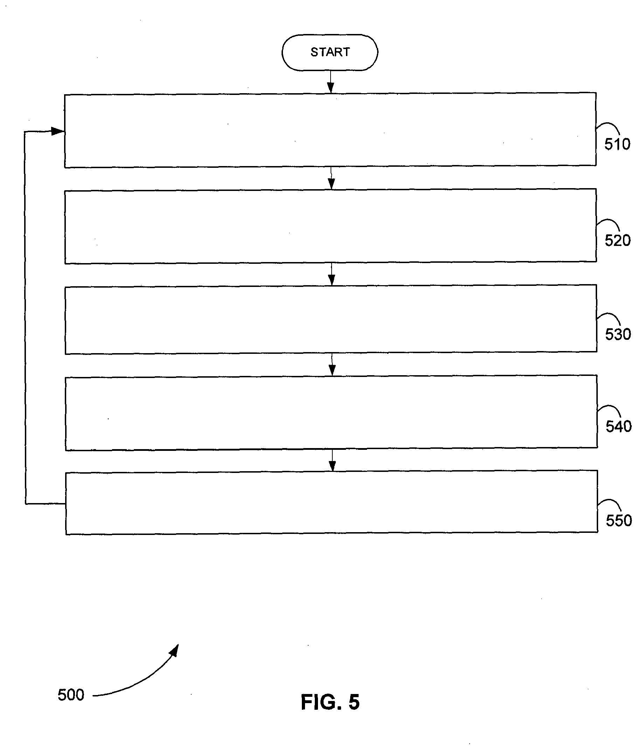

Figure 5 is an exemplary flowchart showing a method 500 for calculating and storing an average amplitude response for each peak of a mass spectrum during data acquisition, in accordance with various embodiments.

In step 510 of method 500, a mass analyzer that includes an analog-to-digital converter (ADC) detector subsystem is instructed to analyze N extractions of an ion beam using a processor. N sub-spectra are produced.

In step 520, for each sub-spectrum of the N sub-spectra, a nonzero amplitude from the ADC detector subsystem is counted as one ion using the processor. A count of one is produced for each ion of each sub-spectrum.

In step 530, the ADC amplitudes and counts of the N sub-spectra are summed using the processor. A spectrum is produced that includes a summed ADC amplitude and a total count for each ion of the spectrum.

In step 540, for each ion of the spectrum, an estimated ion count is calculated from a Poisson distribution of the total count of each ion for the N sub-spectra using the processor.

In step 550, for each ion of the spectrum, an average amplitude response is calculated by dividing the summed amplitude by the estimated ion count and stored using the processor.

Step 510 is executed again using the processor to continue data acquisition.

### Computer Program Product for Calculating and Storing an Average Amplitude Response

In various embodiments, computer program products include a tangible computer-readable storage medium whose contents include a program with instructions being executed on a processor so as to perform a method for calculating and storing an average amplitude response for each peak of a mass spectrum during data acquisition. This method is performed by a system that includes one or more distinct software modules.

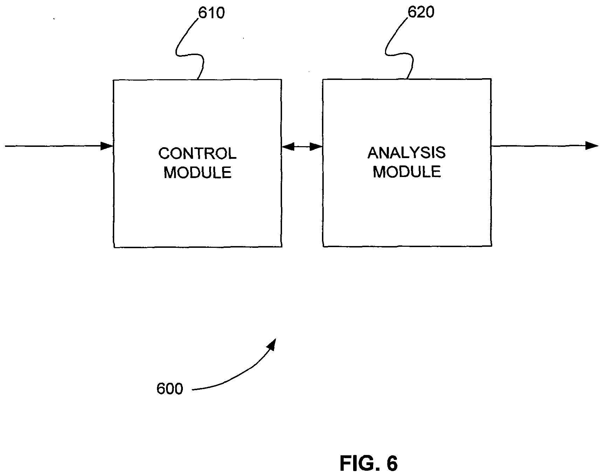

Figure 6 is a schematic diagram of a system 600 that includes one or more distinct software modules that performs a method for calculating and storing an average amplitude response for each peak of a mass spectrum during data acquisition, in accordance with various embodiments. System 600 includes control module 610 and analysis module 620.

Control module 610 instructs a mass analyzer that includes an analog-to-digital converter (ADC) detector subsystem to analyze N extractions of an ion beam. N sub-spectra are produced. For each sub-spectrum of the N sub-spectra, analysis module 620 counts a nonzero amplitude from the ADC detector subsystem as one ion. A count of one for each ion of each sub-spectrum is produced. Analysis module 620 sums the ADC amplitudes and counts of the N sub-spectra. A spectrum is produced that includes a summed ADC amplitude and a total count for each ion of the spectrum. For each ion of the spectrum, analysis module 620 calculates an estimated ion count from a Poisson distribution of the total count of each ion for the N sub-spectra. For each ion of the spectrum, analysis module 620 calculates and stores an average amplitude response by dividing the summed amplitude by the estimated ion count using the analysis module. Finally, control module 610 instructs the mass analyzer to perform another series of N extractions of the sample that producing another N sub-spectra, and the entire process is started again.

While the present teachings are described in conjunction with various embodiments, it is not intended that the present teachings be limited to such embodiments. On the contrary, the present teachings encompass various alternatives, modifications, and equivalents, as will be appreciated by those of skill in the art.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process should not be limited to the performance of their steps in the order written, and one skilled in the art can readily appreciate that the sequences may be varied and still remain within the scope of the appended claims.

## Claims

1. A system (200) for calculating and storing an average amplitude response for each peak of a mass spectrum during data acquisition, comprising:
an ion source that is configured to ionize sample molecules producing a beam of ions;
a mass analyzer (225) that includes an analog-to-digital converter (ADC) detector subsystem (270) configured to analyze the beam of ions; and
a processor (280) in communication with the mass analyzer that is configured to:
(a) instruct the mass analyzer to analyze N extractions of the ion beam, producing N sub-spectra,
(b) for each sub-spectrum of the N sub-spectra, count a nonzero amplitude from the ADC detector subsystem as one ion peak, producing a peak count of one for each ion peak of each sub-spectrum,
(c) sum the ADC amplitudes and peak counts of the N sub-spectra, producing a spectrum that includes a summed ADC amplitude and a total count for each ion peak of the spectrum,
(d) for each ion peak of the spectrum, calculate an estimated ion count from a Poisson distribution of the peak counts of the N sub-spectra,
(e) for each ion peak of the spectrum, calculate and store an average amplitude response by dividing the summed amplitude by the estimated ion count, and
(f) execute step (a) again.

2. The system (200) of claim 1, wherein the mass analyzer (225) further comprises a TDC detector subsystem and the processor (280) is configured to perform step (b) by additionally reading the TDC detector subsystem.

3. The system (200) of any one of the preceding claims, wherein the mass analyzer (225) comprises a quadrupole.

4. The system (200) of any one of the preceding claims, wherein the mass analyzer (225) comprises an ion trap.

5. The system (200) of any one of the preceding claims, wherein the mass analyzer comprises a time-of-flight (TOF) mass analyzer.

6. The system (200) of any one of the preceding claims, wherein for each ion peak of the spectrum, the processor (20) is configured to perform (d) when N exceeds the total count by a threshold level.

7. The system (200) of any one of the preceding claims, wherein an average amplitude response of an ion of the spectrum is used to distinguish the ion from another ion with same mass but a different charge, or to distinguish the ion from another ion with same mass but from an different class of compounds.

8. A method (500) for calculating and storing an average amplitude response for each peak of a mass spectrum during data acquisition, comprising:
(a) instructing (510) a mass analyzer to analyze N extractions of a ion beam using a processor, producing N sub-spectra, wherein the mass analyzer includes an analog-to-digital converter (ADC) detector subsystem and analyzes a beam of ions produced by an ion source that ionizes sample molecules;
(b) for each sub-spectrum of the N sub-spectra, counting (520) a nonzero amplitude from the ADC detector subsystem as one ion peak using the processor, producing a peak count of one for each ion peak of each sub-spectrum;
(c) summing (530) the ADC amplitudes and peak counts of the N sub-spectra using the processor, producing a spectrum that includes a summed ADC amplitude and a total count for each ion peak of the spectrum;
(d) for each ion peak of the spectrum, calculating (540) an estimated ion count from a Poisson distribution of the peak counts of the N sub-spectra using the processor;
(e) for each ion peak of the spectrum, calculating (550) and storing an average amplitude response by dividing the summed amplitude by the estimated ion count using the processor; and
(f) executing step (a) again using the processor.

9. The method of claim 8, wherein step (b) is performed by additionally reading a TDC detector subsystem using the processor.

10. The method (500) of any one of claims 8 or 9, wherein the mass analyzer comprises a quadrupole.

11. The method (500) of any one of claims 8-10, wherein the mass analyzer comprises an ion trap.

12. The method (500) of any one of claims 8-11, wherein the mass analyzer comprises a time-of-flight (TOF) mass analyzer.

13. The method (500)of any one of claims 8-12, wherein (d) is performed when N exceeds the total count by a threshold level.

14. The method (500) of any one of claims 8-13, wherein an average amplitude response of an ion of the spectrum is used to distinguish the ion from another ion with same mass but a different charge, or to distinguish the ion from another ion with same mass but from an different class of compounds.

15. A computer program product, comprising a non-transitory and tangible computer-readable storage medium whose contents include a program with instructions being executed on a processor so as to perform a method for calculating and storing an average amplitude response for each peak of a mass spectrum during data acquisition, the method comprising:
(a) providing a system (600), wherein the system comprises one or more distinct software modules, and wherein the distinct software modules comprise a control module (610) and an analysis module (620);
(b) instructing a mass analyzer to perform a series of N extractions of a beam of ions using the control module, producing N sub-spectra, wherein the mass analyzer includes an analog-to-digital converter (ADC) detector subsystem and analyzes a beam of ions produced by an ion source that ionizes sample molecules;
(c) for each sub-spectrum of the N sub-spectra, counting a nonzero amplitude from the ADC detector subsystem as one ion peak using the analysis module, producing a peak count of one for each ion peak of each sub-spectrum;
(d) summing the ADC amplitudes and peak counts of the N sub-spectra using the analysis module, producing a spectrum that includes a summed ADC amplitude and a total count for each ion peak of the spectrum;
(e) for each ion peak of the spectrum, calculating an estimated ion count from a Poisson distribution of the peak counts of for the N sub-spectra using the analysis module;
(f) for each ion peak of the spectrum, calculating and storing an average amplitude response by dividing the summed amplitude by the estimated ion count using the analysis module; and
(g) executing step (a) again using the control module.

## Patentansprüche

1. System (200) zum Berechnen und Speichern einer durchschnittlichen Amplitudenantwort für jeden Höchstwert eines Massenspektrums während der Datenerfassung, wobei das System Folgendes umfasst:
eine Ionenquelle, die dafür konfiguriert ist, Probenmoleküle zu ionisieren, die einen Ionenstrahl erzeugen;
einen Massenanalysator (225), der ein Analog-Digital-Wandler- (Analog-to-Digital Converter, ADC) Detektorsubsystem (270) umfasst, das dafür konfiguriert ist, den Ionenstrahl zu analysieren; und
einen Prozessor (280) in Kommunikation mit dem Massenanalysator, der für Folgendes konfiguriert ist:
(a) Anweisen des Massenanalysators, N Extraktionen des Ionenstrahls zu analysieren, wobei N Subspektren erzeugt werden,
(b) Zählen für jedes Subspektrums der N Subspektren einer Amplitude ungleich Null aus dem ADC-Detektorsubsystem als einen einzelnen Ionenhöchstwert, wobei für jeden Ionenhöchstwert jedes Subspektrums eine Höchstwertzählung von eins erzeugt wird,
(c) Summieren der ADC-Amplituden und Höchstwertzählungen der N-Subspektren, wobei ein Spektrum erzeugt wird, das eine summierte ADC-Amplitude und eine Gesamtzählung für jeden Ionenhöchstwert des Spektrums umfasst,
(d) Berechnen für jeden Ionenhöchstwert des Spektrums einer geschätzten Ionenzählung aus einer Poisson-Verteilung der Höchstwertzählungen der N Subspektren,
(e) Berechnen und Speichern für jeden Ionenhöchstwert des Spektrums einer durchschnittlichen Amplitudenantwort, indem die summierte Amplitude durch die geschätzte Ionenzählung dividiert wird, und
(f) erneutes Ausführen von Schritt (a).

2. System (200) nach Anspruch 1, wobei der Massenanalysator (225) ferner ein TDC-Detektorsubsystem umfasst und der Prozessor (280) dafür konfiguriert ist, Schritt (b) durch zusätzliches Lesen des TDC-Detektorsubsystems auszuführen.

3. System (200) nach einem der vorhergehenden Ansprüche, wobei der Massenanalysator (225) ein Quadrupol umfasst.

4. System (200) nach einem der vorhergehenden Ansprüche, wobei der Massenanalysator (225) eine Ionenfalle umfasst.

5. System (200) nach einem der vorhergehenden Ansprüche, wobei der Massenanalysator einen Flugzeit- (time-of-flight, TOF) Massenanalysator umfasst.

6. System (200) nach einem der vorhergehenden Ansprüche, wobei für jeden Ionenhöchstwert des Spektrums der Prozessor (20) dafür konfiguriert ist, (d) auszuführen, wenn N die Gesamtzählung um einen Schwellenwert überschreitet.

7. System (200) nach einem der vorhergehenden Ansprüche, wobei eine durchschnittliche Amplitudenantwort eines Ions des Spektrums verwendet wird, um das Ion von einem anderen Ion mit derselben Masse, aber einer anderen Ladung zu unterscheiden, oder um das Ion von einem anderen Ion mit derselben Masse, aber von einer anderen Klasse von Verbindungen zu unterscheiden.

8. Verfahren (500) zum Berechnen und Speichern einer durchschnittlichen Amplitudenantwort für jeden Höchstwert eines Massenspektrums während der Datenerfassung, wobei das Verfahren Folgendes umfasst:
(a) Anweisen (510) eines Massenanalysators, N Extraktionen eines Ionenstrahls unter Verwendung eines Prozessors zu analysieren, wobei N Subspektren erzeugt werden, wobei der Massenanalysator ein Analog-Digital-Wandler- (ADC) Detektorsubsystem umfasst und einen Ionenstrahl analysiert, der von einer Ionenquelle erzeugt wird, die Probenmoleküle ionisiert;
(b) für jedes Subspektrum der N Subspektren, Zählen (520) einer Amplitude ungleich Null vom ADC-Detektorsubsystem als ein Ionenhöchstwert unter Verwendung des Prozessors, wodurch eine Höchstwertzählung von eins für jeden Ionenhöchstwert jedes Subspektrums erzeugt wird;
(c) Summieren (530) der ADC-Amplituden und Höchstwertzählungen der N Subspektren unter Verwendung des Prozessors, Erzeugen eines Spektrums, das eine summierte ADC-Amplitude und eine Gesamtzählung für jeden Ionenhöchstwert des Spektrums umfasst;
(d) Berechnen (540), für jeden Ionenhöchstwert des Spektrums, einer geschätzten Ionenzählung aus einer Poisson-Verteilung der Höchstwertzählungen der N-Subspektren unter Verwendung des Prozessors;
(e) Berechnen (550) und Speichern, für jeden Ionenhöchstwert des Spektrums, einer durchschnittlichen Amplitudenantwort durch Teilen der summierten Amplitude durch die geschätzte Ionenzählung unter Verwendung des Prozessors; und
(f) erneutes Ausführen von Schritt (a) unter Verwendung des Prozessors.

9. Verfahren nach Anspruch 8, wobei Schritt (b) durch zusätzliches Lesen eines TDC-Detektorsubsystems unter Verwendung des Prozessors ausgeführt wird.

10. Verfahren (500) nach einem der Ansprüche 8 oder 9, wobei der Massenanalysator ein Quadrupol umfasst.

11. Verfahren (500) nach einem der Ansprüche 8 bis 10, wobei der Massenanalysator eine Ionenfalle umfasst.

12. Verfahren (500) nach einem der Ansprüche 8 bis 11, wobei der Massenanalysator einen Flugzeit-Massenanalysator (TOF) umfasst.

13. Verfahren (500) nach einem der Ansprüche 8 bis 12, wobei (d) durchgeführt wird, wenn N die Gesamtzählung um einen Schwellenwert überschreitet.

14. Verfahren (500) nach einem der Ansprüche 8 bis 13, wobei eine durchschnittliche Amplitudenantwort eines Ions des Spektrums verwendet wird, um das Ion von einem anderen Ion mit derselben Masse, aber einer anderen Ladung zu unterscheiden, oder um das Ion von einem anderen Ion mit derselben Masse, aber von einer anderen Klasse von Verbindungen zu unterscheiden.

15. Computerprogrammprodukt, das ein nicht-flüchtiges und greifbares computerlesbares Speichermedium umfasst, dessen Inhalt ein Programm mit Anweisungen umfasst, die auf einem Prozessor ausgeführt werden, um ein Verfahren zum Berechnen und Speichern einer durchschnittlichen Amplitudenantwort für jeden Höchstwert eines Massenspektrums während der Datenerfassung durchzuführen, wobei das Verfahren Folgendes umfasst:
(a) Bereitstellen eines Systems (600), wobei das System ein oder mehrere verschiedene Softwaremodule umfasst, und wobei die verschiedenen Softwaremodule ein Steuermodul (610) und ein Analysemodul (620) umfassen;
(b) Anweisen eines Massenanalysators, eine Reihe von N Extraktionen eines Ionenstrahls unter Verwendung des Steuermoduls durchzuführen, wobei N Subspektren erzeugt werden; wobei der Massenanalysator ein Analog-Digital-Wandler- (ADC) Detektorsubsystem umfasst und einen Ionenstrahl analysiert, der von einer Ionenquelle erzeugt wird, die Probenmoleküle ionisiert;
(c) Zählen, für jedes Subspektrum der N Subspektren, einer Amplitude ungleich Null vom ADC-Detektorsubsystem als ein Ionenhöchstwert unter Verwendung des Analysemoduls; wodurch eine Höchstwertzählung von eins für jeden Ionenhöchstwert jedes Subspektrums erzeugt wird;
(d) Summieren der ADC-Amplituden und Höchstwertzählungen der N Subspektren unter Verwendung des Analysemoduls, Erzeugen eines Spektrums, das eine summierte ADC-Amplitude und eine Gesamtzählung für jeden Ionenhöchstwert des Spektrums umfasst;
(e) Berechnen, für jeden Ionenhöchstwert des Spektrums, einer geschätzten Ionenzählung aus einer Poisson-Verteilung der Höchstwertzählungen für die N-Subspektren unter Verwendung des Analysemoduls;
(f) Berechnen und Speichern, für jeden Ionenhöchstwert des Spektrums, einer durchschnittlichen Amplitudenantwort durch Teilen der summierten Amplitude durch die geschätzte Ionenzählung unter Verwendung des Analysemoduls; und
(g) erneutes Ausführen von Schritt (a) unter Verwendung des Steuermoduls.

## Revendications

1. Système (200) pour calculer et stocker une réponse d'amplitude moyenne pour chaque pic d'un spectre de masse pendant l'acquisition de données, comprenant :
une source d'ions qui est configurée pour ioniser des molécules d'échantillon produisant un faisceau d'ions ;
un analyseur de masse (225) qui comprend un sous-système de détection de convertisseur analogique-numérique (ADC) (270) configuré pour analyser le faisceau d'ions ; et
un processeur (280) en communication avec l'analyseur de masse qui est configuré pour :
(a) ordonner à l'analyseur de masse d'analyser N extractions du faisceau d'ions, produisant N sous-spectres,
(b) pour chaque sous-spectre des N sous-spectres, compter une amplitude non nulle du sous-système de détection ADC comme un pic ionique, produisant un comptage de pics de un pour chaque pic ionique de chaque sous-spectre,
(c) additionner les amplitudes ADC et le nombre de pics des N sous-spectres, produisant un spectre qui comprend une amplitude ADC additionnée et un comptage total pour chaque pic ionique du spectre,
(d) pour chaque pic ionique du spectre, calculer un comptage d'ions estimé à partir d'une distribution de Poisson des comptages de pics des N sous-spectres,
(e) pour chaque pic ionique du spectre, calculer et stocker une réponse d'amplitude moyenne en divisant l'amplitude additionnée par le comptage d'ions estimé, et
(f) exécuter à nouveau l'étape (a).

2. Système (200) selon la revendication 1, dans lequel l'analyseur de masse (225) comprend en outre un sous-système de détecteur TDC et le processeur (280) est configuré pour exécuter l'étape (b) en lisant en plus le sous-système de détecteur TDC.

3. Système (200) selon l'une quelconque des revendications précédentes, dans lequel l'analyseur de masse (225) comprend un quadripôle.

4. Système (200) selon l'une quelconque des revendications précédentes, dans lequel l'analyseur de masse (225) comprend un piège à ions.

5. Système (200) selon l'une quelconque des revendications précédentes, dans lequel l'analyseur de masse comprend un analyseur de masse à temps de vol (TOF).

6. Système (200) selon l'une quelconque des revendications précédentes, dans lequel pour chaque pic ionique du spectre, le processeur (20) est configuré pour effectuer (d) lorsque N dépasse le comptage total d'un niveau de seuil.

7. Système (200) selon l'une quelconque des revendications précédentes, dans lequel une réponse d'amplitude moyenne d'un ion du spectre est utilisée pour distinguer l'ion d'un autre ion avec la même masse mais avec une charge différente, ou pour distinguer l'ion d'un autre ion avec la même masse mais appartenant à une classe de composés différente.

8. Procédé (500) pour calculer et stocker une réponse d'amplitude moyenne pour chaque pic d'un spectre de masse pendant l'acquisition de données, comprenant :
(a) l'instruction (510) à un analyseur de masse d'analyser N extractions d'un faisceau d'ions à l'aide d'un processeur, produisant N sous-spectres, dans lequel l'analyseur de masse comprend un sous-système de détection de convertisseur analogique-numérique (ADC) et analyse un faisceau d'ions produit par une source d'ions qui ionise des molécules d'échantillon ;
(b) pour chaque sous-spectre des N sous-spectres, le comptage (520) d'une amplitude non nulle du sous-système de détection ADC comme un pic ionique à l'aide du processeur, produisant un comptage de pics de un pour chaque pic ionique de chaque sous-spectre ;
(c) l'addition (530) des amplitudes ADC et des comptages de pics des N sous-spectres en utilisant le processeur, produisant un spectre qui comprend une amplitude ADC additionnée et un comptage total pour chaque pic ionique du spectre ;
(d) pour chaque pic ionique du spectre, le calcul (540) d'un comptage d'ions estimé à partir d'une distribution de Poisson des comptages de pics des N sous-spectres en utilisant le processeur ;
(e) pour chaque pic ionique du spectre, le calcul (550) et le stockage d'une réponse d'amplitude moyenne en divisant l'amplitude additionnée par le comptage d'ions estimé à l'aide du processeur ; et
(f) l'exécution à nouveau de l'étape (a) en utilisant le processeur.

9. Procédé selon la revendication 8, dans lequel l'étape (b) est effectuée en lisant en plus un sous-système de détection TDC à l'aide du processeur.

10. Procédé (500) selon l'une quelconque des revendications 8 ou 9, dans lequel l'analyseur de masse comprend un quadripôle.

11. Procédé (500) selon l'une quelconque des revendications 8 à 10, dans lequel l'analyseur de masse comprend un piège à ions.

12. Procédé (500) selon l'une quelconque des revendications 8 à 11, dans lequel l'analyseur de masse comprend un analyseur de masse à temps de vol (TOF).

13. Procédé (500) selon l'une quelconque des revendications 8 à 12, dans lequel (d) est effectuée lorsque N dépasse le comptage total d'un niveau de seuil.

14. Procédé (500) selon l'une quelconque des revendications 8 à 13, dans lequel une réponse d'amplitude moyenne d'un ion du spectre est utilisée pour distinguer l'ion d'un autre ion avec la même masse mais avec une charge différente, ou pour distinguer l'ion d'un autre ion avec la même masse mais d'une classe différente de composés.

15. Produit programme d'ordinateur, comprenant un support de stockage non transitoire et tangible lisible par ordinateur dont le contenu comprend un programme avec des instructions exécutées sur un processeur de manière à exécuter un procédé de calcul et de stockage d'une réponse d'amplitude moyenne pour chaque pic d'un spectre de masse pendant l'acquisition de données, le procédé comprenant :
(a) la fourniture d'un système (600), dans lequel le système comprend un ou plusieurs modules logiciels distincts, et dans lequel les modules logiciels distincts comprennent un module de commande (610) et un module d'analyse (620) ;
(b) l'instruction à un analyseur de masse d'effectuer une série de N extractions d'un faisceau d'ions en utilisant le module de commande, produisant N sous-spectres, l'analyseur de masse comprenant un sous-système de détection de convertisseur analogique-numérique (ADC) et analyse un faisceau d'ions produit par une source d'ions qui ionise les molécules d'échantillon ;
(c) pour chaque sous-spectre des N sous-spectres, le comptage d'une amplitude non nulle du sous-système de détection ADC comme un pic ionique en utilisant le module d'analyse, produisant un comptage de pic de un pour chaque pic ionique de chaque sous-spectre;
(d) l'addition des amplitudes ADC et des comptages de pics des N sous-spectres en utilisant le module d'analyse, produisant un spectre qui comprend une amplitude ADC additionnée et un comptage total pour chaque pic ionique du spectre ;
(e) pour chaque pic ionique du spectre, le calcul d'un comptage d'ions estimé à partir d'une distribution de Poisson des comptages de pics pour les N sous-spectres en utilisant le module d'analyse ;
(f) pour chaque pic ionique du spectre, le calcul et le stockage d'une réponse d'amplitude moyenne en divisant l'amplitude additionnée par le nombre d'ions estimé en utilisant le module d'analyse ; et
(g) l'exécution à nouveau de l'étape (a) en utilisant le module de commande.
